(19) Europäisches Patentamt
European Patent Office
Office européen des brevets

(11) Publication number: 0 398 540 A1

(12) # EUROPEAN PATENT APPLICATION

(21) Application number: 90304720.7

(22) Date of filing: 01.05.90

(51) Int. Cl.[5]: C07K 13/00, C12N 15/30, A61K 39/015

(30) Priority: 03.05.89 US 346863

(43) Date of publication of application: 22.11.90 Bulletin 90/47

(84) Designated Contracting States: AT BE CH DE DK ES FR GB GR IT LI LU NL SE

(71) Applicant: SMITHKLINE BEECHAM CORPORATION
P.O. Box 7929 1 Franklin Plaza
Philadelphia Pennsylvania 19101(US)

(72) Inventor: Gross, Mitchell Stuart
667 Pugh Road
Wayne, Pennsylvania 19087(US)
Inventor: Young, james Francis
12624 Gravenhurst Lane
Gaithersburg, MD 20878(US)

(74) Representative: Giddings, Peter John, Dr. et al
SmithKline Beecham, Corporate Patents, Mundells
Welwyn Garden City, Hertfordshire AL7 1EY(GB)

(54) **Malaria vaccine.**

(57) A malaria vaccine comprising a vaccinal polypeptide having one or more immunogenic determinants from a first region flanking a central repeat domain of a Plasmodium surface protein, one or more immunogenic determinants from a second region flanking the repeat domain and fewer than all or none of the repeating immunogenic determinants from the central repeat domain.

EP 0 398 540 A1

# MALARIA VACCINE

## BACKGROUND OF THE INVENTION

### 1. Field of the Invention

This invention relates to vaccines against infection by parasites of the genus Plasmodium and, more particularly, to polypeptides useful as therapeutic agents to inhibit infection by malaria parasites, which polypeptides comprise immunogenic determinants from regions of a Plasmodium surface protein flanking a central repeat domain thereof and fewer than all repeating immunogenic determinants from the repeat domain; to methods for purifying these polypeptides; to expression vectors encoding these polypeptides; and to methods for treating humans against malaria infection.

### 2. Description of the Related Art

Malaria is a severe, widespread disease for which, despite years of extensive efforts, a vaccine has not been developed. See, for example, Science, Volume 226, page 679 (November 9, 1984). Experimentally, mammals, including man, have been protected against infection by the etiologic agent of malaria, Plasmodium, by vaccination with irradiated sporozoites. Clyde et al., Am. J. Trop. Med. Hyg., Volume 24, page 397 (1975) and Rieckman et al., Bull, WHO, Volume 57 (Supp. 1), page 261 (1979). Yoshida et al., Science, Volume 207, page 71 (1980) report that such protection is at least partially mediated by antibody directed against a protein on the surface of the sporozoite, the circumsporozoite (CS) protein; monoclonal antibodies raised against CS proteins neutralize infectivity in vitro and protect animals in vivo. The CS protein appears to be highly evolutionarily conserved within species, but is quite varied across species.

Four species of Plasmodium are known to infect man. These are P. falciparum, P. vivax, P. ovale and P. malariae, the latter two occurring at much lower frequency. Other species of scientific interest are P. berghei and P. knowlesi, the hosts of these species being respectively, rodents and monkeys.

Kemp et al., WO.84-02917-A, disclose cloning and expression of P. falciparum cDNA in E. coli.

Dame et al., Science, Volume 225, page 593 (1984), report cloning and expression of the CS protein of P. falciparum in E. coli. The protein is described as comprising about 412 amino acids with an approximate molecular weight of 44,000. It comprises 41 tandem repeats of a tetrapeptide. Synthetic 7-, 11- and 15-residue peptides derived from the repeat region bound to monoclonal antibodies raised against the CS protein.

Antisporozoite vaccines based upon the repeating tetrapeptides of the CS protein of P. falciparum have not been successful, conferring immunity in few individuals and that immunity was of short duration. Science 241:522 (1988). Consequently, the need for an effective vaccine against malaria parasite remains unfilled.

## SUMMARY OF THE INVENTION

This invention relates generally to a polypeptide comprising one or more immunogenic determinants from a first region flanking a central repeat domain of a Plasmodium surface protein, one or more immunogenic determinants from a second region flanking the repeat domain and fewer than all or none of the repeating immunogenic determinants from the central repeat domain.

In one embodiment, the invention relates to a polypeptide comprising at least one but fewer than all repeating immunogenic determinants of a Plasmodium surface protein repeat domain and one or more immunogenic determinants from regions of a Plasmodium surface protein flanking the repeat domain.

In another embodiment of the invention, the polypeptide comprises substantially all of the immunogenic determinants from the regions flanking the central repeat domain, and is devoid of immunogenic determinants from the central repeat domain. Alternatively, the polypeptide comprising substantially all immunogenic determinants from the flanking regions further comprises at least one but fewer than all immunogenic determinants from the central repeat domain.

Immunogenic determinants useful in the polypeptides of the present invention preferably include those present in the surface proteins of Plasmodium falciparum, P. vivax, P. malariae, and P. ovale.

In yet another embodiment of the invention, the polypeptide is genetically fused to a carrier protein, preferably a carrier protein which either enhances expression of the polypeptide or enhances the immunogenicity of the polypeptide, or both.

In a preferred embodiment, the polypeptides of the present invention comprise an immunogenic carrier protein, for example, 81 N-terminal amino acids of influenza virus nonstructural protein 1 ($NS1_{81}$), fused, via a synthetic linker, to a first flanking region of a Plasmodium circumsporozoite (CS) protein, which is itself fused to a second flanking region of the CS protein.

Such polypeptides may further comprise more than one but fewer than all immunogenic determinants from the CS protein central repeat domain, for example, the immunogenic determinant from the repeat domain comprising a tetrapeptide having the amino acid sequence (Asn-X-Y-Pro), wherein X is Ala or Val and Y is Asn or Asp. The immunogenic determinants from the central repeat domain may be positioned, for example, between the carrier protein and the first flanking region or between the first flanking region and the second flanking region.

Another aspect the present invention includes expression vectors encoding the polypeptides, vaccines comprised of the polypeptides; methods for purifying the polypeptides; and methods for treating humans against infection by malaria using the peptides.

Other aspects and advantages of the present invention are disclosed in the detailed description which follows.

## BRIEF DESCRIPTION OF THE DRAWINGS

Figures 1(a) and 1(b) present ELISA data in graph form demonstrating the antibody response of two groups of C3H/HEN mice, one group immunized with $NS1_{81}RLf\Delta9$, and the other group immunized with $R32tet_{32}$;

Figures 2(a) and 2(b) present ELISA data in graph form demonstrating the antibody response of two groups of C57BL/6 mice, one group immunized with $NS1_{81}RLf\Delta9$ and the other group immunized with $R32tet_{32}$; and

Figures 3(a) and 3(b) present ELISA data in graph form demonstrating the antibody response of two groups of BALB/C mice, one group immunized with $NS1_{81}RLf\Delta9$ and the other group immunized with $R32tet_{32}$.

## DESCRIPTION OF THE PREFERRED EMBODIMENTS

The protozoan malaria parasite, Plasmodium, has a number of stage-specific proteins present on its outer cell surface. These surface proteins have been found to have three regions in common, namely, a central repeated epitope region or domain and paired flanking regions. The first flanking region of the pair is fused to the carboxy-terminus of the central repeat domain and the second flanking region of the pair is fused to the amino terminus of the central repeat domain.

The polypeptides of the present invention are derived from a portion of a Plasmodium surface protein which contains fewer than all or none of the immunogenic determinants of the central repeat domain between the first and second flanking regions and are expressed in quantities sufficient for use as therapeutic agents to inhibit infection by malaria parasites.

In other words, the polypeptides of the invention have fewer tandem repeats between the first and second flanking regions than are present in the wild-type repeat regions. Thus, in the case of a polypeptide for protecting a human against infection by P. falciparum, the polypeptide may comprise the entire first flanking region, that is, the N-terminal flanking region of the P. falciparum circumsporozoite protein (PfCSP), the entire second flanking region, that is, the C-terminal flanking region from the PfCSP and, between the first and second flanking regions, less than 41 tandem repeats from the PfCSP, that is, less than 41 tetrapeptides of the formula Asn-X-Y-Pro, wherein X is Ala or Val and Y is Asp or Asn.

In a wild-type Plasmodium surface protein, the repeat region is immunodominant. In the polypeptides of the present invention, the number and positioning of tandem repeats, or repeating units, is selected so as not to mask the immune response to the first and second flanking regions. Preferably, the number of repeats in the polypeptides of the invention is no more than one-half of the number of repeats present in

the wild-type protein. More preferably, the number of repeats in the polypeptides of the invention is no more than about one quarter of the number of repeats present in the wild-type protein.

Four species of Plasmodium are known to infect man, the most prevalent being P. falciparum followed by P. vivax and, to a lesser extent, P. malariae and P. ovale.

The central repeat domain of the Plasmodium falciparum sporozoite stage circumsporozoite (CS) protein is comprised of 41 tandemly repeating tetrapeptides, thirty one of which have the amino acid sequence (Asparagine (Asn)-Alanine (Ala)-Asn-proline (Pro) and four of which have the sequence (Asn-Valine (Val)-Aspartate (Asp)-Pro). On either side the central repeat domain are the so-called flanking regions containing Region I and Region II, two regions of the CS protein nearly identical in amino acid sequence to the corresponding regions of P. knowlesi (a monkey malaria) CS protein (Dame et al., Science, 225:593 (1984)).

Various surface proteins of P. falciparum blood stages also have repeated epitopes, for example, S antigen (Pro-Ala-Lys-Ala-Ser-Gln-Gly-Gly-Leu -Glu-Asp); RESA antigen (Glu-Glu-Asn-Val-Glu-His-Asp-Ala); FIRA antigen (Val-Thr-Thr-Gln-Glu-Pro); and PF-11 antigen (Glu-Glu-Val-Val-Glu-Glu-Val-Val-Pro).

The circumsporozoite protein of the P. vivax contains the repeated epitope (Gly-Asp-Arg-Ala-Asp-Gly-Gln-Pro-Ala) and the circumsporozoite protein of P. malariae contains the repeated epitope (Asn-Asp-Ala-Gly) and (Asn-Ala-Ala-Gly).

The polypeptides of the present invention comprise one or more immunogenic determinants from a first region flanking the central repeat domain of a Plasmodium surface protein, one or more immunogenic determinants from a second region flanking the repeat domain and fewer than all or none of the repeating immunogenic determinants from the central repeat domain.

Immunogenic determinants are amino acid sequences which elicit a B cell or T cell response. Immunogenic determinants generally comprise at least 6 amino acids. Precise identification of immunogenic determinants within a protein can be made by standard techniques involving monoclonal antibody mapping and/or deletion of amino acids followed by activity assay. Preferably, the polypeptides of the invention comprises at least about 15 amino acids from each of the first and second flanking regions; more preferably, at least about 30; and, most preferably, the entire first and second flanking regions, less the signal sequence.

In one embodiment, the polypeptides of the present invention comprise at least one but fewer than all immunogenic determinants from a Plasmodium surface protein central repeat domain and one or more immunogenic determinants from regions of the surface protein flanking the repeat domain.

In another embodiment, the polypeptides of the present invention comprise substantially all of the immunogenic determinants from the regions flanking the central repeat domain and are devoid of immunogenic determinants from the central repeat domain or, alternatively, contain at least one but fewer than all immunogenic determinants from the central repeat domain. By "substantially all" is meant that substantially the entire first and second flanking regions, less the signal sequence, are employed; preferably, no more than about 20 amino acids are lacking from each region, and, more preferably, the entire first and second flanking regions, less the signal sequence, are employed.

Preferably, the polypeptides of the present invention are hybrid polypeptides, that is, proteins comprised of the genetic fusion between a portion of the surface protein and a carrier protein.

More preferred are hybrid polypeptides which include a carrier protein genetically fused to a portion of the Plasmodium falciparum circumsporozoite (CS) protein containing fewer than all or devoid of the repeating tetrapeptides which comprise the central repeat domain.

Particularly preferred are those hybrid polypeptides in which the carrier protein not only enhances immunogencity of the carried polypeptide but which also enhances expression of the polypeptide by a transformant. Other desirable properties of such carrier proteins include enhancing purification or formulation of the polypeptide. Examples of such carrier proteins include $NS1_{81}$ (81 N-terminal amino acids of influenza virus (A/PR/8/34) non-structural protein 1) (Baez et al., Nucleic Acids Research, 8:5845 (1980)); R32 ([Asn-Ala-Asn-Pro$)_{15}$- (Asn-Val-Asp-Pro)]$_2$) (Young et al., Science, 228:958 (1985)); and galK.

Specific embodiments of the types of polypeptides of the present invention exemplified herein include:

$NS1_{81}$RLfΔ9, a fusion polypeptide comprising 81 N-terminal amino acids of influenza virus non-structural protein 1 ($NS1_{81}$); the Region I-containing flanking region of the P. falciparum CS protein less the signal sequence (18 N-terminal amino acids) and the Region II-containing flanking region less the first nine N-terminal amino acids thereof (RLfΔ9). Fusion of $NS1_{81}$ to RLfΔ9 is facilitated through a synthetic DNA linker sequence encoding-Asp-His-Met-Leu-Thr-Asp-;

$NS1_{81}$-RLfAuth, a fusion polypeptide comprising $NS1_{81}$; the Region I-containing flanking region of the P. falciparum CS protein less the signal sequence and the entire Region II-containing flanking region (RLfAuth);

$NS1_{81}$-(Asn-X-Y-pro)$_n$-RLfAuth, a fusion polypeptide comprising $NS1_{81}$; RLfAuth, and (Asn-X-Y-Pro), wherein X is Ala or Val and Y is Asn or Asp and n is an integer greater than or equal to one and less than or equal to 100, preferably less than 50; and, further, wherein the (Asn-X-Y-Pro) is positioned between $NS1_{81}$ and RLfAuth; and

$NS1_{81}$RLfAuth+(Asn-X-Y-Pro)$_n$, a fusion polypeptide comprising $NS1_{81}$; RLfAuth; and (Asn-X-Y-Pro) wherein X and Y are defined as above and n is < 41; and, further, wherein (Asn-X-Y-Pro) is positioned between the Region I-containing flanking region of the P. falciparum CS protein and the Region II-containing flanking region, that is, the region formerly occupied by the central repeat domain.

Such polypeptides are, however, illustrative only. Based on the disclosure provided herein, one skilled in the art will know how to construct and test other polypeptides within the scope of the invention, for example, polypeptides comprising sequences from surface proteins, including circumsporozoite protein of the various malaria parasites other than P. falciparum, polypeptides comprising more or fewer amino acids from the surface proteins, polypeptides which are chemically modified, and polypeptides which are fused to other or additional amino acid or protein sequences. Such polypeptides are readily constructed by standard techniques of genetic engineering and/or protein synthesis and can be tested in animal models substantially as described hereinbelow. For example, a protein of the invention may comprise amino acid sequences from surface protein flanking regions, such as substantially the entire circumsporozoite protein devoid of the central repeat domain, fused to the surface antigen of Hepatitis B Virus (HBsAg) in a fusion protein which can form hybrid HBsAg particles, as described in European patent application EP 278,940 published August 17, 1988.

A genetic coding sequence for surface protein flanking regions, tetrapeptides, synthetic DNA linker sequences, and carrier proteins can be easily obtained by one skilled in the art using known techniques. These include synthesis and, preferably, by reverse transcription of messenger RNA or by direct cloning of intact genes from genomic DNA. Reverse transcription of P. falciparum messenger RNA is described in Ellis et al., Nature, 302:536 (1963). Direct cloning of intact genes from P. falciparum genomic DNA is described in Dame et al., (cited above). Cloning and expression of repeat-containing polypeptides is described in copending application EP-191 748-A, the disclosure of which is incorporated herein by reference.

Having cloned all or a portion of Plasmodium DNA, fragments thereof encoding all or a portion of the surface protein can be prepared by known techniques.

Techniques for synthesizing DNA are well known and may be accomplished using commercially available DNA synthesizers.

Coding sequences for polypeptides may be inserted into E. coli expression vectors, many of which are known and readily available. In carrying out the present invention in E. coli, a DNA sequence which encodes the polypeptide of the present invention is operatively linked to a regulatory element within a DNA vector for transformation in E. coli. Numerous gram negative bacterial expression vectors comprising such regulatory elements are available. The regulatory element comprises a promoter which effect RNA polymerase binding and transcription. Regulatable, that is, inducible or derepressable, promoters are preferred. A variety of useful promoters and available for expression of heterologous polypeptides in E. coli. These include the trp promoter and the lambda PL promoter (eg. U.S. Patent No. 4.578.355 and Courtney et al., Nature, 313:149 (1985). As described in more detail below, it has been found that coding sequences encoding the polypeptides of the present invention are particularly well expressed by the E. coli expression vector pMG-1. Derivatives of pMG-1, encoding carrier proteins other than $NS1_{81}$, for example, R32 and galK, may also be used to advantage.

In carrying out the present invention in Streptomyces, a DNA coding sequence which encodes the polypeptide of the present invention is operatively linked to a regulatory element within a DNA vector for transformation of Streptomyces. The regulatory element comprises a promoter which effects RNA polymerase binding and transcription. Regulatable, i.e., inducible or derepressible, promoters are preferred. A variety of useful promoters are available for exprssion of heterologous polypeptides in Streptomyces. Examples include the galactose-inducible promoter of the Streptomyces galactose operon (Fornwald, et al., Proc Natl. Acad. Sci. USA 84:2130 (1987)), the constitutive promoter of the S. lividans β-galactosidase gene (Eckhardt, et al. J. Bacteriol. 169:4249 (1987); Brawner, et al., U.S. Patent 4,717,666) and the S. longisporus trypsin inhibitor gene (European Patent Application No. 87 307 260.7), or a temporally regulated promoter such as that reported in M. echinosporsa (Baum, et al., J. Bacteriol 170:71 (1988)). Regions for transcription termination in Streptomyces are derived from the 3' end of several Streptomyces genes, for example the termination signal at the end of the Streptomyces galactose operon or that found at the end of the S. fradiae neomycin phosphotransferase gene (Thompson and Gray, Proc. Natl. Acad. Sci USA 80:5190 (1983)). Sequences for protein export in Streptomyces include those isolated from the S. lividans β-galactosidase

gene, the S. lividans LEP-10 gene (European Patent Application No. 87 307 260.7) and the S. longisporus trypsin inhibitor gene.

The gene encoding the polypeptide of the present invention is incorporated into a larger DNA molecule which comprises a genetic selection marker system. The selection marker system can be any of a number of known marker systems such that the marker gene confers a selectable new phenotype on the transformed cell. Examples include Streptomyces drug resistance genes such as thiostrepton resistance ribosomal methylase (Thompson, et al., Gene 20:51 (1982)), neomycin phosphotransferase (Thompson, et al., supra) and erthromycin resistance ribosomal methylase (Thompson, et al., supra). The DNA molecule may also contain a sequence for autonomous replication in Streptomyces, such as the pIJ101 derivatives (Keiser, et al., Mol. Gen. Genet. 185:223 (1982)) or an SLP1 derived vector (Bibb, et al., Mol. Gen. Genet. 184:230 (1981)). The DNA molecule may also contain a marker which permits gene amplification. Such markers which serve to amplify gene copy number in Streptomyces include the gene for spectinomycin resistance (Hornemann, et al., J. Bacteriol 169:2360 (1987)) and arginine auxotrophy (Altenbuchner, et al., Mol. Gen. Genet. 195:134 (1984)).

In carrying out the present invention in yeast, a DNA coding sequence which encodes the polypeptides of the present invention is operatively linked to a regulatory element within a DNA vector for transformation of yeast. Any yeast host for which transformation, cloning and expession systems are available can be used. Particular examples include yeasts of the genera Hansenula, Pichia, Kluveromyces, Schizosaccharomyces, Candida and Saccharomyces. The preferred yeast host is Saccharomyces cerevisiae.

The regulatory element comprises a promoter which effects RNA polymerase binding and transcription. Regulatable, i.e., inducible or derepressible, promoters are preferred. A variety of useful promoters are available for expression of heterologous polypeptides in yeast. These include the copper inducible metallothionine gene (CUP1) promoter and the constitutive promoter of the glycolytic genes glyceraldehye-3 phosphate dehydrogenase (TDH3) and alcohol dehydrogenase (ADH). Regions for transcriptional termination in yeast are derived from the 3′ end of any of several yeast genes, for example the gene for iso-1-cytochrome C (CYC1)

The gene encoding the polypeptide of the present invention is incorporated into a larger DNA molecule which comprises a genetic selection marker system. The selection marker system can be any of a number of known marker systems, such that the marker gene confers a selectable new phenotype on the transformed cell. Examples include yeast genes for biosynthetic enzymes such as phospho-ribosyl anthranilate isomerase (TRP1) or orotidine-5′-phosphate decarboxylase (URA3) or heterologous drug resistance genes such as G418 resistance or benomyl anthranilate isomerase (TRP1) or benomyl resistance (BEN1). The DNA molecule may also contain a sequence for autonomous replication in yeast, such as the yeast 2-micron-circle ori region or a chromosomal autonomous replication region (ARS), such as ARS1, and a yeast centromere (CEN), such as CEN3, to allow for autonomous replication of the plasmid.

Still other expression systems are known and readily available. For example, a variety of insect cells and expression systems therefor are available for expression of heterologous proteins, such as a baculovirus expression system for use in expressing heterologous proteins in Lepidoptera cells. Where necessary to effect expression in eukaryotic expression systems, it may be necessary to delete the carboxy terminal anchor region of the surface protein. By way of example, deletion of amino acids 392-412, the sequence encompassing the P. falciparum carboxy terminal anchor region may be required. (See PCT/US 89/05550, filed 13 December 1989, the disclosure of which is incorporated herein by reference.)

Another exemplary expression system is that disclosed in EP-357 208-A, the disclosure of which is incorporated herein by reference, which relates to a Salmonella bacterial strain transformed with a selected heterologous gene operatively linked to an E. coli promoter sequence, the transformant being capable of constitutively expressing the product of the heterologous gene.

The polypeptides so expressed are isolated and purified from the producing cell culture using standard protein isolation techniques, many of which are well known in the art. An exemplary, useful purification scheme comprises (1) the disruption of the bacterial cells, (2) clarification of cellular debris, (3) separation of the polypeptides of the present invention from other polypeptides present in the clarified cell extract, and (4) final purification to remove residual contaminants, including residual polypeptides, carbohydrates, nucleic acids, lipopolysaccharides and endotoxins.

In the vaccine of the invention, an aqueous solution of the polypeptide, preferably buffered at physiological pH, can be used directly. Alternatively, the polypeptides can be admixed or absorbed with any of a number of known adjuvants. Such adjuvants include, for example, aluminum hydroxide, muramyl dipeptide and saponons such as Quil A. As a further example, the polypeptide may be encapsulated within microparticles such as liposomes. In yet another alternative, the polypeptides of tee present invention may be conjugated to an immunostimulating macromolecule, such as killed Bordetella or a tetanus toxoid.

Vaccine preparations are generally described in New Trends and Developments in Vaccines, Voller et al., Eds., University Park Press, Baltimore, MD, USA (1978). Encapsulation within liposomes is described, for example, in U.S. Patent No. 4,235,877 to Fullerton. Conjugation of proteins to macromolecules is disclosed in U.S. Patent No. 4,372,945 to Likhite and U.S. Patent No. 4,474,757 to Armor et al. Use of Quil A is disclosed, for example, by Dalsgaard et al., Acta Vet. Scand., 18:349 (1977).

The amount of polypeptide present in each vaccine dose is that amount which induces an immunoprotective response without significant, adverse side effects. Such amounts will vary according to the specific polypeptide employed and whether or not the vaccine is adjuvanted. Generally, it is expected that each dose will comprise 1 to 1000 ug. of polypeptide, preferably 10 to 200 ug. An optimal amount for a particular vaccine can be ascertained by standard studies involving observation of antibody titers and other responses in subjects. Following an initial vaccination, subjects will preferably receive a boost in about four weeks, followed by additional boosts every six months for as long as the risk of infection exists.

Intramuscular, subcutaneous or intravenous administrations are generally preferred, although in some cases, other routes may be useful. For example, where recombinant Salmonella are employed, the preferred route of administration may be oral.

The following Examples are illustrative, and not limiting, of the invention. The CS protein coding sequence was supplied by James Weber, Walter Reed Army Institute for Research, as a 2337 base pair EcoR I fragment of λmPF1 (Dame et al., Science 225:593 (1984)) in the EcoR I site of pUC8, a standard E. coli cloning vector (available, for example, from Bethesda Research Laboratories, Inc., Gaithersburg, MD). The resulting pUC8 derivative is referred to as pUC8 clone 1.

## EXAMPLE 1

### Construction of $pNS1_{81}RLf\Delta9$

Briefly summarized, construction of $pNS1_{81}RLf\Delta9$ was completed as follows. A first aliquot of pCSP (described below), an E. coli expression vector containing a 1216 base pair fragment encoding all but the first 18 amino acids of the P. falciparum circumsporozoite (CS) protein, was digested with restriction endonuclease Fok I, end-filled (Klenow Fragment) and digested with restriction endonuclease BamH I. The resulting 318 base pair fragment, encoding amino acids 19 (Leu) to 123 (Pro) of the CS protein, was recovered by electroelution.

A second aliquot of pCSP was digested with restriction endonuclease Tth111 I, end-filled and digested with restriction endonuclease Sal I. The resulting 655 base pair fragment, encoding amino acids 297 (Gly) to 412 (Asn) of the CS protein, was recovered by electroelution. (This sequence is lacking 9 N-terminal amino acids, Nos. 288 (Pro) to 296 (Gln), of the Region II-containing flanking region.)

The 318 base pair and 655 base pair CS protein gene fragments were ligated into E. coli expression vector pUC18 (described below) previously digested with restriction endonuclease BamH I and Sal I. The resulting vector was named pUCRLfΔ9.

pUCRLfΔ9 was digested with restriction endonuclease BamH I, end-filled, and digested with restriction endonuclease Sal I. The resulting 1035 base pair fragment, encoding CS protein amino acids 19 to 123 and 297 to 412, was recovered by electroelution.

Expression vector pMG-1 (described below), containing a DNA fragment encoding N-terminal amino acids 1 (Met) to 81 (Met) of influenza virus non-structural protein 1 and a synthetic DNA linker sequence, was digested with restriction endonuclease EcoR V and Xho I. The 1035 base pair fragment, previously isolated from pUCRLfΔ9, was then ligated into pMG-1. The resulting expression vector, $pNS1_{81}RLf\Delta9$ encodes a protein having the following sequence:

$NS1_{81}$-Asp-His-Met-Leu-Thr-Asp-Pro-$CS_{19-123}$-$CS_{297-412}$.

Construction of $pNS1_{81}RLf\Delta9$ is detailed below.

#### A. Construction of pCSP

Purified pUC8 clone 1 plasmid DNA (40 µg.) was digested with restriction endonucleases Stu I and Rsa I (100 units of each enzyme) in 400 ul. of medium buffer (comprising 50 mM Tris, 50 mM NaCl, 1 mM

dithiothreitol (DTT), and 10 mM $MgCl_2$, having a pH of 7.5) for 1.5 hours at 37° C. The resulting 1216 base pair fragment, encoding all but the first 18 amino acids (believed to encode the CS protein signal sequence) of the circumsporozoite (CS) protein, was isolated by electrophoresis on a 6% polyacrylamide gel (PAGE) and recovered by electroelution.

Ten micrograms of expression vector pAS1 (ATCC 39262, more fully described in U.S. Patent No. 4,578,355 to M. Rosenberg) was digested with restriction endonuclease BamH I (25 units) in 200 µl. medium buffer (described above) for 1.5 hours at 37° C. The cut plasmid was then treated for 15 minutes at 25° C with DNA Polymerase I, Large Fragment (5 units of Klenow Fragment in 20 mM Tris-HCl, pH 7.5, 7 mM $MgCl_2$, 60 mM NaCl, 6 mM 2-mercaptoethanol and 0.25 mM of each of the four deoxynucleotide triphosphates to end-fill the BamH I site).

The circumsporozoite protein gene fragment (1 µg.) was then ligated into this vector (100 ng.) in 30 µl. ligase buffer (comprising 50 mM Tris, 1 mM DTT, 10 mM $MgCl_2$ 100 µM rATP, having a pH of 7.5) with one unit of T4-DNA ligase for 16 hours at 4° C.

The ligation mixture was transformed into E. coli strain MM294CI+. Ampicillin resistant colonies were obtained and screened for insertion of the CS gene fragment into pAS1. A plasmid with the correct construction (pCSP) was identified.

B. Construction of pUCRLfΔ9

Purified pCSP plasmid DNA (100 µg.) was digested with restriction endonuclease Fok I (100 units) in 400 µl. of medium buffer (described above) for 3 hours at 37° C. Subsequently, the plasmid was treated with DNA Polymerase I, Large Fragment (described above) to end-fill the Fok I site. The plasmid was next digested with restriction endonuclease BamH I (100 units) in 400 µl. of medium buffer (described above) for 3 hours at 37° C. The resulting 318 base pair fragment, encoding amino acids 19-123 of the CS protein, was isolated by electrophoresis on a 6% polyacrylamide gel (PAGE) and recovered by electroelution.

An additional aliquot (100 µg.) of pCSP was digested with restriction endonuclease Tth111 I (100 units) in 400 µl. of medium buffer (described above) for 3 hours at 65° C. Subsequently, the plasmid was treated with DNA Polymerase I, Large Fragment (described above) to fill in the Tth111 I site. The plasmid was next digested with restriction endonuclease Sal I (100 units) in 400 µl. of medium buffer for 3 hours at 37° C and the resulting 655 base pair fragment, encoding amino acids 297-412 of the CS protein, was isolated by electrophoresis on a 6% polyacrylamide gel and recovered by electroelution.

Ten micrograms of expression vector pUC18 (Yanish-Perron et al., Gene, 33:103 (1985)), a standard E. coli cloning vector, (available, for example, from Bethesda Research Laboratories, Inc., Gaithersburg, MD) was digested with restriction endonuclease BamH I and Sal I (20 units each) in 200 µl. of medium buffer (described above) for 2 hours at 37° C. The 318 base pair BamH I end-filled/Fok I fragment (1 µg.) and the 655 base pair Tth111 I end-filled/Sal I fragment (1 µg.) was then ligated into pUC18 in 30 µl. ligase buffer (described above) with one unit of T4 - DNA ligase for 16 hours at 4° C. A plasmid with the correct construction (pUCRLfΔ9) was identified.

C. Construction of pMG-1

Ten micrograms of expression vector pMG27N- (M.Gross et al., Mol. Cell. Biol., 5:1015 (1985)) was digested with restriction endonucleases BamH I and Sac I (50 units of each) in 200 µl. medium buffer (described above) for 3 hrs. at 37° C.

Ten micrograms of expression vector pAPR801 (Young et al., Proc. Natl, Acad. Sci. U.S.A., 80:6105 (1983)) containing the influenza virus (A/PR/8/34) non-structural protein 1 (NSI) coding region (Baez, et al., Nucleic Acids Research, 8:5845 (1980)) was digested with restriction endonucleases NcoI and BamH I (20 units each) in 200 µl. of high buffer (50 mM Tris-HCl, 1 mM DTT, 10 mM $MgCl_2$, and 100 mM NaCl, pH of 7.5) for 2 hours at 37° C. The resulting 230 base pair fragment, encoding the first 81 N-terminal amino acids of NS1, was isolated by electrophoresis on a 6% polyacrylamide gel (PAGE) and recovered by electroelution.

Forty nanograms of the BamH I/Sac I-cut pMG27N-(described above) was ligated with 80 ng. of the 230 base pair Nco I/BamH I $NS1_{81}$-encoding fragment and 80 ng. of a synthetic linker having the following sequence:

```
                   Asp His Met Leu Thr Asp
BamHI 5'CATGGATCATATGTTAACAGATATCAAGGCCTGACTGACTGAGAGCT 3'  SacI
      3'     CTAGTATACAATTGTCTATAGTTCCGGACTGACTGACTC        5'
```

The resulting plasmid, pMG-1, was identified with the BamH I site of the $NS1_{81}$ encoding sequence ligated to the BamH I site of pMG27N-; the Nco I site of the $NS1_{81}$ encoding sequence ligated to the Nco I site of the synthetic linker; and the Sac I site of the synthetic linker ligated to the Sac I site of pMG27N-. This vector introduces unique restriction sites to facilitate insertion of DNA fragments in any of three reading frames, results in the insertion of TGA termination codons in all three reading frames downstream of the ATG initiation codon of the cII ribosome binding site and, when expressed, results in $NS1_{81}$ fusion proteins from all three reading frames. Digestion of pMG-1 with restriction endonuclease Nde I and subsequent ligation of the vector as described above results in the expression of a non-fusion protein (that is, not fused to $NS1_{81}$).

D. Construction of $pNS1_{81}RLf\Delta9$

Expression vector pUCRLf (100 µg.) was digested with restriction endonuclease BamH I (100 units) in 400 µl. high buffer (described above) for 3 hours at 37°C. The cut plasmid was subsequently treated with DNA Polymerase I, Large Fragment (described above) to end-fill the BamH I site. The plasmid was next digested with restriction endonuclease Sal I (20 units) in 400 µl. medium buffer (described above) for 3 hours at 37°C. The resulting 1035 base pair fragment was isolated by electrophoresis on a 6% polyacrylamide gel (PAGE) and recovered by electroelution.

Expression vector pMG-1 (10 µg.) was digested with restriction endonucleases EcoR V and Xho I (25 units of each) in 400 µl. of medium buffer (described above) for 3 hours at 37°C. The 1035 base base pair BamH I end-filled/ Sal I (400 µg.) fragment from pUCRLf was then ligated into this vector (100 ng.) in 30 ul. of ligase buffer (described above) with one unit of T4-DNA ligase for 16 hours at 4°C.

The ligation mixture was transformed into E. coli strain MM294C1+. Ampicillin resistant colonies were obtained and screened for clones containing the properly oriented inserted gene. A plasmid with the correct construction ($pNS1_{81}RLf\Delta9$) was identified, transformed in E. coli strain AR58 (CIts857) and tested for expression of the circumsporozoite protein gene product devoid of the first 18 N-terminal amino acids ($CS_{1-18}$), the central repeat domain, and 9 N-terminal amino acids ($CS_{248-296}$) of the Region II-containing flanking region ($RLf\Delta9$), fused, via 6 amino acids (Asp-His-Met-Leu-Thr-Asp) derived from the synthetic linker ligated into the pMG-1 expression vector, to 81 N-terminal amino acids of the influenza non-structural protein 1, $NS1_{81}$. $NS1_{81}RLf\Delta9$ has the following sequence:

$NS1_{81}$-Asp-His-Met-Leu-Thr-Asp-Pro-$CS_{19-123}$-$CS_{297-412}$.

The proline separating the Asp (from the C terminus of the synthetic linker) from $RLf\Delta9$ ($CS_{19-123}$-$CS_{297-412}$) is an artifact of the filled-in BamH I site of the BamH I/Fok I fragment of pCSP.

Cells were grown in Luria-Bertani Broth (LB) at 32°C to an absorbance at 650nm ($A_{650}$) of 0.6 and temperature induced at 42°C for 3 hours to turn on transcription of the PL promoter of the expression plasmid and subsequent translation of the $NS1_{81}$ CS protein derivative. Cells were sampled in 1 ml. aliquots, pelleted, resuspended in lysis buffer (10 mM Tris-HCl, pH 7.8, 25% (vol/vol) glycerol, 2% 2-mercaptoethanol, 2% sodium dodecyl sulfate (SDS), 0.1% bromophenyl blue) and incubated in a 105°C heating block for 5 minutes.

Proteins were separated by SDS-PAGE (13% acrylamide, 30:0.8 acrylamide:bis-acrylamide ratio). Proteins were transferred to nitrocellulose and the $NS1_{81}RLf\Delta9$ protein produced in E. coli was detected by Western Blot analysis using polyclonal antibodies reactive with a domain of the CS protein called Region I (Dame et al, Science 225:593 (1984)) as well as polyclonal antibodies reactive with $NS1_{81}$ protein. The E. coli produced $NS1_{81}RLf\Delta9$ protein was also shown to be non-reactive with a pool of 5 monoclonal antibodies directed to the tetrapeptide repeat domain of the P. falciparum CS protein.

## EXAMPLE 2

Construction of $pNS1_{81}RLfAuth$

E. coli expression vector pCSP (described above) was digested with restriction endonuclease BamH I and Fok I in medium buffer. The resulting DNA fragment, encoding the Region I-containing flanking region, less the first 18 N-terminal amino acids, was recovered by electroelution.

A second aliquot of pCSP was digested with restriction endonuclease Tth111 I and Sal I in medium buffer. The resulting DNA fragment, encoding the Region II-containing flanking region, less the first 9 N-terminal amino acids, was recovered by electroelution.

E. coli expression vector pUC18 (described above) was digested with restriction endonuclease BamH I and Sal I in medium buffer.

To restore the 9 N-terminal amino acids ($CS_{288-296}$) of the Region II - containing region of the C-terminal flank of the central repeat domain of the CS protein, which amino acids were lost in the digestion of pCSP with restriction endonuclease Tthi111 I, a synthetic DNA fragment containing a Fok I end and a Tth111 I end, and having the following sequence, was prepared:

```
            Asp Pro Gly Asn Lys Asn Asn Gln Gly Asn Gly Gln
Fok I    5'  ATCCCGGGAATAAAAACAACCAAGGTAATGGACA  3'   Tth 111 I
         3'      GCCCTTATTTTTGTTGGTTCCATTACCTGTT  5'

         The BamH I/Fok I fragment   Tth111 I/Sal I fragment
```

The BamH I/Fok I fragment, Tth111 I/Sal I fragment and the synthetic fragment were ligated into the BamH I/Sal I digested pUC18.

The resulting plasmid, pUCRLfAuth, was digested with restriction endonuclease BamH I in medium buffer, end-filled, and digested with restriction endonuclease Sal I. The resulting DNA fragment, encoding authentic circumsporozoite protein lacking the first 18 N-terminal amino acids and the central repeat domain, was recovered by electroelution.

The isolated BamH I end-filled/Sal I fragment was then ligated into the $NS1_{81}$-encoding E. coli expression vector, pMG-1 (described above), which had previously been digested with restriction endonuclease EcoR V and Xho I. The resulting expression vector, $pNS1_{81}$RLfAuth, expresses a protein having the sequence:

$NS1_{81}$-Asp-His-Met-Leu-Thr-Asp-Pro-$CS_{19-123}$-Gly-$CS_{299-412}$

(The glycine separating the Region I and Region II-containing CS flanking regions ($CS_{19-123}$ and $CS_{288-412}$) is an artifact of the synthetic Fok I/Tth111 I DNA linker sequence.

The complete nucleotide and amino acid sequence for $NS1_{81}$RLFAuth is given below:

```
     NSI
     ATggatccaaacactgtgtcaagctttcaggtagattgctttctttggcatgtccgcaaa
   1 ---------+---------+---------+---------+---------+---------+ 60
     TAcctaggtttgtgacacagttcgaaagtccatctaacgaaagaaaccgtacaggcgttt

     MetAspProAsnThrValSerSerPheGlnValAspCysPheLeuTrpHisValArgLys -

     cgagttgcagaccaagaactaggtgatgccccattccttgatcggcttcgccgagatcag
  61 ---------+---------+---------+---------+---------+---------+ 120
     gctcaacgtctggttcttgatccactacggggtaaggaactagccgaagcggctctagtc

     ArgValAlaAspGlnGluLeuGlyAspAlaProPheLeuAspArgLeuArgArgAspGln -

     aaatccctaagaggaaggggcagcactcttggtctggacatcgagacagccacacgtgct
 121 ---------+---------+---------+---------+---------+---------+ 180
     tttagggattctccttccccgtcgtgagaaccagacctgtagctctgtcggtgtgcacga

     LysSerLeuArgGlyArgGlySerThrLeuGlyLeuAspIleGluThrAlaThrArgAla -

     ggaaagcagatagtggagcggattctgaaagaagaatccgatgaggcacttaaaatgacc
 181 ---------+---------+---------+---------+---------+---------+ 240
     cctttcgtctatcacctcgcctaagactttcttcttaggctactccgtgaattttactgg

     GlyLysGlnIleValGluArgIleLeuLysGluGluSerAspGluAlaLeuLysMetThr -

                  Linker           CS
     atggatcatatgttacagatcccTTATTCCAGGAATACCAGTGCTATGGAAGTTCGTCA
 241 ---------+---------+---------+---------+---------+---------+ 300
     tacctagtatacaattgtctagggAATAAGGTCCTTATGGTCACGATACCTTCAAGCAGT
     81                        19
     MetAspHisMetLeuThrAspProLeuPheGlnGluTyrGlnCysTyrGlySerSerSer -

     AACACAAGGGTTCTAAATGAATTAAATTATGATAATGCAGGCACTAATTTATATAATGAA
 301 ---------+---------+---------+---------+---------+---------+ 360
     TTGTGTTCCCAAGATTTACTTAATTTAATACTATTACGTCCGTGATTAAATATATTACTT

     AsnThrArgValLeuAsnGluLeuAsnTyrAspAsnAlaGlyThrAsnLeuTyrAsnGlu -

     TTAGAAATGAATTATTATGGGAAACAGGAAAATTGGTATAGTCTTAAAAAAAATAGTAGA
 361 ---------+---------+---------+---------+---------+---------+ 420
     AATCTTTACTTAATAATACCCTTTGTCCTTTTAACCATATCAGAATTTTTTTTATCATCT

     LeuGluMetAsnTyrTyrGlyLysGlnGluAsnTrpTyrSerLeuLysLysAsnSerArg -

     TCACTTGGAGAAAATGATGATGGAAATAATAATAATGGAGATAATGGTCGTGAAGGTAAA
 421 ---------+---------+---------+---------+---------+---------+ 480
     AGTGAACCTCTTTTACTACTACCTTTATTATTATTACCTCTATTACCAGCACTTCCATTT

     SerLeuGlyGluAsnAspAspGlyAsnAsnAsnAsnGlyAspAsnGlyArgGluGlyLys -
```

```
     GATGAAGATAAAAGAGATGGAAATAACGAAGACAACGAGAAATTAAGGAAACCAAAACAT
481  ---------+---------+---------+---------+---------+---------+ 540
     CTACTTCTATTTTCTCTACCTTTATTGCTTCTGTTGCTCTTTAATTCCTTTGGTTTTGTA

     AspGluAspLysArgAspGlyAsnAsnGluAspAsnGluLysLeuArgLysProLysHis -

     AAAAAATTAAAGCAACCAGGGGATGGTAATCCTGATCCCgggaataaaaacaaccaaggt
541  ---------+---------+---------+---------+---------+---------+ 600
     TTTTTTAATTTCGTTGGTCCCCTACCATTAGGACTAGGGcccttatttttgttggttcca

     LysLysLeuLysGlnProGlyAspGlyAsnProAspProGlyAsnLysAsnAsnGlnGly -

     aatggacaaGGTCACAATATGCCAAATGACCCAAACCGAAATGTAGATGAAAATGCTAAT
601  ---------+---------+---------+---------+---------+---------+ 660
     ttacctgttCCAGTGTTATACGGTTTACTGGGTTTGGCTTTACATCTACTTTTACGATTA

     AsnGlyGlnGlyHisAsnMetProAsnAspProAsnArgAsnValAspGluAsnAlaAsn -

     GCCAACAATGCTGTAAAAAATAATAATAACGAAGAACCAAGTGATAAGCACATAGAACAA
661  ---------+---------+---------+---------+---------+---------+ 720
     CGGTTGTTACGACATTTTTTATTATTATTGCTTCTTGGTTCACTATTCGTGTATCTTGTT

     AlaAsnAsnAlaValLysAsnAsnAsnAsnGluGluProSerAspLysHisIleGluGln -

     TATTTAAAGAAAATAAAAAATTCTATTTCAACTGAATGGTCCCCATGTAGTGTAACTTGT
721  ---------+---------+---------+---------+---------+---------+ 780
     ATAAATTTCTTTTATTTTTTAAGATAAAGTTGACTTACCAGGGGTACATCACATTGAACA

     TyrLeuLysLysIleLysAsnSerIleSerThrGluTrpSerProCysSerValThrCys -

     GGAAATGGTATTCAAGTTAGAATAAAGCCTGGCTCTGCTAATAAACCTAAAGACGAATTA
781  ---------+---------+---------+---------+---------+---------+ 840
     CCTTTACCATAAGTTCAATCTTATTTCGGACCGAGACGATTATTTGGATTTCTGCTTAAT

     GlyAsnGlyIleGlnValArgIleLysProGlySerAlaAsnLysProLysAspGluLeu -

     GATTATGAAAATGATATTGAAAAAAAAATTTGTAAAATGGAAAAATGTTCCAGTGTGTTT
841  ---------+---------+---------+---------+---------+---------+ 900
     CTAATACTTTTACTATAACTTTTTTTTTAAACATTTTACCTTTTTACAAGGTCACACAAA

     AspTyrGluAsnAspIleGluLysLysIleCysLysMetGluLysCysSerSerValPhe -

     AATGTCGTAAATAGTTCAATAGGATTAATAATGGTATTATCCTTCTTGTTCCTTAATTAG
901  ---------+---------+---------+---------+---------+---------+ 960
     TTACAGCATTTATCAAGTTATCCTAATTATTACCATAATAGGAAGAACAAGGAATTAATC

     AsnValValAsnSerSerIleGlyLeuIleMetValLeuSerPheLeuPheLeuAsnEnd -

     ATAA
961  ---- 964
     TATT
```

## EXAMPLE 3

Construction of $pNS1_{81}RLfAuth + (NANP)_2$

$pNS1_{81}$RLFAuth (described above) was digested with restriction endonuclease Sma I in medium buffer (described above and containing KCl) at 25° C for 3 hours.

A synthetic DNA linker, having the sequence:

```
   AsnAlaAsnProAsnAlaAsnPro
5' AACGCAAACCCAAATGCAAACCCC 3'
3' TTGCCTTTGGGTTTACGTTTGGGG 5'
```

was ligated into the Sma I-digested $pNS1_{81}$RLfAuth. The synthetic DNA linker encodes $(NANP)_2$ (single letter symbols designating amino acids, N = asparagine (Asn); A = alanine (Ala); and P = proline (Pro)), the tetrapeptide comprising the so-called consensus sequence of the immunodominant repeat domain of the CS protein. Digestion of $pNS1_{81}$RLfAuth with restriction endonuclease Sma I permits ligation of any number of repeating tetrapeptides encoded by a synthetic DNA linker into that region of the CS protein formerly occupied by the immunodominant repeat domain. Additional tetrapeptide-encoding DNA fragments may be ligated into the vector in this manner. The resulting plasmid, $pNS1_{81}$RLfAuth + $(NANP)_2$ encodes a protein having the sequence:

$NS1_{81}$-Asp-His-Met-Leu-Thr-Asp-Pro-$CS_{19-123}$-$(NANP)_2$-Gly-$CS_{288-412}$

## EXAMPLE 4

### Construction of $pNS1_{81}(NANP)_4$RLfAuth

Expression vector pUCRLfAuth (described above) was digested with restriction endonuclease BamH I.

A synthetic DNA fragment, encoding $(NANP)_4$ was ligated into the BamH I digested pUCRLf. The synthetic DNA fragment had the following sequence:

```
       Pro Asn Ala Asn Pro Asn Ala Asn Pro Asn Ala Asn Pro Asn Ala Asn Pro
5 ' GATCCCAATGCAAACCCAAATGCAAACCCAAACGCTAACCCCAACGCTAACCCC        3'
3'         GGTTACGTTTGGGTTTACGTTTGGGTTTGCGATTGGGGTTGCGATTGGGGCTAG 5'
```

The resulting plasmid was named $pUC18(NANP)_4$RLfAuth

Expression vector $pUC18(NANP)_4$RLfAuth was digested with restriction endonuclease BamH I, end-filled (Klenow Fragment), digested with restriction endonuclease Sal I, and the resulting DNA base pair fragment covered by electroelution.

The BamH I end-filled/Sal I fragment was ligated into the $NS1_{81}$-encoding expression vector pMG1 (described above), previously digested with restriction endonuclease EcoR V and Xho I. The resulting plasmid was named $pNS1_{81}(NANP)_4$RLfAuth and encodes a protein wherein repeating tetrapeptides encoded by the synthetic DNA fragment are inserted between amino acid 81 (Met) of $NS1_{81}$ and N-terminal Asp of the Nco I/Sac I synthetic DNA linker:

$NS1_{81}(NANP)_4$-Asp-His-Met-Leu-Thr-Asp-Pro-$CS_{19-123}$-Gly-$CS_{288-412}$

## EXAMPLE 5

### Construction of $pNS1_{81}(NVDP)_4$RLfAuth

Construction of $pNS1_{81}(NVDP)_4$RLfAuth was the same as that described above for $pNsl_{81}(NANP)$-$_4$RLfAuth except that the synthetic DNA linker, encoding $(NVDP)_4$ (the variant tetrapeptide sequence of the CS protein central repeat domain), had the following sequence:

```
          Asn Val Asp Pro Asn Val Asp Pro Asn Val Asp Pro Asn Val
5'  GATCCCAATGTAGACCCCAACGTTGATCCGAACGTAGACCCGAATGTA  3'
3'        GGTTACATCTGGGGTTGCAACTAGGCTTGCATCTGGGCTTACAT  5'
```

The resulting plasmid encodes a protein having the sequence:
$NS1_{81}(NVDP)_4$-Asp-His-Met-Leu-Thr-Asp-Pro-$CS_{19-123}$-Gly$CS_{288-412}$

## EXAMPLE 6

### Isolation of $NS1_{81}$RLfΔ9 from *E. coli*

Following the induction of synthesis of $NS1_{81}$RLfΔ9 in a temperature sensitive lambda lysogen (CIts857), the bacterial cells were collected by centrifugation and the resulting pellet frozen at -70° C. Approximately 12 g. of the concentrated and frozen cells were thawed by dilution in 120 ml. of a lysis buffer solution (pH 8) containing 50 mM Tris(hydroxymethyl) aminomethane (TRIS), 10 mM ethylenediaminetetraacetic acid (EDTA), 5% glycerol and 10 mM dithiothreitol (DTT). Lysozyme (Sigma Chemical Co., St. Louis, MO) was added to a final concentration of 0.2 mg./ml. of diluted cells and the solution stirred at 4° C for 30 minutes. The cells were sonicated using a Branson sonicator until the solution appeared liquified. A 10% deoxycholate solution was added to a final concentration of 0.1% (v/v) and the solution was centrifuged at 15,600 x G for 30 minutes at 4° C in a Sorvall RC 5B centrifuge (Dupont).

The supernatant was discarded and the remaining protein-containing pellet was suspended in 100 ml. of a buffer solution (pH 10) containing 50 mM glycine, 2 mM EDTA, and 5% glycerol. The suspension was sonicated as described above and Triton® X-100 (Sigma) added to a final concentration of 1% (v/v). The sonicated solution was stirred at 4° C for 30 minutes and centrifuged as described above.

Urea was added to the protein-containing supernatant to a final urea concentration of 8 M and the sample titrated to pH 5.5 with a 50% solution of acetic acid. The sample was chromatographed on a 25 ml. column of QAE Sepharose® Fast Flow (Pharmacia) previously equilibrated in a buffer solution (pH 5.5) containing 20 mM sodium acetate, 1% Triton X-100, and 8 M urea, at a flow rate of 300 cm./hr. The protein was in the unbound fraction and applied to a 10 ml. column of SP Sepharose® Fast Flow (Pharmacia) previously equilibrated in a buffer solution (pH 5.5) containing 20 mM sodium acetate and 8M urea, at a flow rate of 120 cm./hr. The effluent was monitored for absorbance at 280 nm. The protein was eluted from this column with a buffer (pH 8) containing 100 mM Tris and 8 M urea.

SDS-PAGE of the resulting product revealed a major band with an apparent Mr of 40,000 kD, and of approximately 80% purity. The purification process yielded 12 mg. of protein containing approximately 14 endotoxin units/mg. of protein.

## EXAMPLE 7

### Isolation of $NS1_{81}$RLfΔ9 from *E. coli*

Following the induction of synthesis of $NS1_{81}$RLfΔ9 in a temperature sensitive lambda lysogen, the bacterial cells were collected by centrifugation and the resulting pellet frozen at -70° C. Approximately 636 g. of the concentrated and frozen cells were thawed by dilution in 2200 ml. of a buffer solution (pH 8.0) containing 60 mM Tris, 12 mM EDTA, 6% glycerol and 12 mM dithiothreitol (DTT). The thawed cells were passed through a Manton Gaulin homogenizer two times at 6000-7000 psi. A 10% deoxycholate solution was added to a final concentration of 0.1% (v/v), the lysate stirred at 4° C for 30 minutes and centrifuged at 10,000 x G in a Sorvall RC 58 centrifuge (Dupont) at 4° C for 60 minutes.

The pellet was discarded and 25 µl. of 1050 or 2100 Biocryl bead mixture (Supelco) was added to each ml. of the protein-containing supernatant. The solution was stirred and centrifuged as described above.

The pellet was discarded and solid ammonium sulfate was added over a five minute period to the remaining protein-containing supernatant to 20% saturation.

The sample was stirred and centrifuged as described above. The pellet was suspended in 400 ml. of a buffer solution (pH 5.5) containing 20 mM sodium acetate, 1% Triton® X-100 and 8 M urea. This suspension was centrifuged and the supernatant was dialyzed against a buffer solution (pH 8.0) containing 100 mM Tris and 8 M urea. The retentate was adjusted to pH 5.5 and chromatographed on a 100 ml. column of SP Sepharose®, Fast Flow (Pharmacia) previously equilibrated with a buffer solution (pH 5.5) containing 20 mM sodium acetate, 1% Triton® X-100 and 8 M urea at a flow rate of 10 ml./min. The column was washed with equilibration buffer, followed by a buffer containing 0.1 M Tris and 8 M urea at pH 8. The column was eluted with a 500 ml. linear gradient of 0.0 to 0.5 M sodium chloride prepared in a buffer (pH 8) containing 0.1 M Tris and 8 M urea.

The protein eluted at 0.3 M sodium chloride. The fractions containing the product were pooled and concentrated in an Amicon stirred cell using a YM 10 membrane and dialyzed against a buffer solution (pH 8.0) containing 20 mM Tris, followed by dialysis against phosphate buffered saline (pH 7.0).

SDS-PAGE analysis of the resulting product revealed a major band with an apparent Mr of 40,000 kD and a series of minor components. The purification process yielded 25 mg. of protein containing 144 endotoxin units/mg. protein.

## EXAMPLE 8

### Isolation of R16CSP from E. coli

R16 CSP, expressed in E. coli, was prepared by ligating an Xho II fragment isolated from pUC8 clone 1 (described above) into pCSP (described above) previously digested with restriction endonuclease BamH I. R16CSP, used as an ELISA capture antigen in Example 9 below, was purified as follows.

Following the induction of synthesis of R16CSP in E. coli, the bacterial cells were collected by centrifugation and the resulting pellet frozen at -20° C. Approximately 373 g. of the concentrated and frozen cells was thawed in 1.43 l. of buffer (pH 3.0) containing 50 mM Tris, 10 mM EDTA, 5% glycerol, and 10 mM dithiothreitol. A 10% deoxycholate (w/v) solution was added to a final concentration of 0.1% (v/v), after which the cells were twice passed through a Manton-Gaulin homogenizer at 7,000 psi. A 10% solution of polyethyleneimine (BRL) in 0.5 M Tris, pH 8.0 buffer (w/v) was added to the homogenate to a final concentration of 0.5%. The solution was stirred at 4° C for 1 hour and centrifuged at 13,000 x g in a Sorvall RC 2B centrifuge (Dupont) at 4° C for 45 minutes.

The pellet was discarded and solid ammonium sulfate was added to the supernatant to a saturation of 35% over 5 minutes. The solution was stirred and centrifuged as previously described.

The pellet was suspended in a buffer (pH 8.0) containing 300 ml. of 20 mM Tris, and 10 mM EDTA. A solution containing 300 ml. of 8 M urea, 2.7 l. of 10 mM sodium acetate, and 4 M urea were added and the pH was adjusted to 4.0 immediately. The sample was centrifuged as described previously.

The supernatant was applied to a 50 ml. column of SP-Sepharose®, Fast Flow (Pharmacia) equilibrated in a buffer (pH 4.0) containing 20 mM sodium acetate and 4 M urea. The column was washed with a buffer (pH 5.0) containing 20 mM sodium acetate and eluted with a 250 ml. linear gradient containing 0.0 to 1.0 M sodium chloride in wash buffer. The product eluted at approximately 0.3 M sodium chloride.

A 50 ml. aliquot of the ion exchange product was adjusted to pH 2.3 with 10% trifluoroacetic acid (TFA)(v/v) and chromatographed on a C4 reverse phase column (Vydac, 1 X 25 cm.) equilibrated in 0.1% TFA. A linear gradient of 0 to 60% acetonitrile (ACN) in 0.1% TFA was run over 45 minutes and the product eluted at approximately 60% ACN. The reverse phase product was neutralized by the addition of 25 ul./ml. of 1 M ammonium bicarbonate.

Approximately 45 ml. of the reversed phase product was dialyzed against a buffer (pH 5.0) containing 2 M guanidine hydrochloride, and concentrated to 20 ml. on a YM 30 membrane (Amicon). A 10 ml. aliquot was chromatographed in a 2.5 X 50 cm. column of Superose® 12 (Pharmacia) equilibrated in a buffer (pH 5.0) containing 2.0 M guanidine hydrochloride. The protein which eluted at an apparent Mr of 358kD was dialyzed against a buffer (pH 4.5) containing 20 mM sodium acetate.

Coomassie stained SDS-PAGE of the Superose product revealed two major bands with an apparent Mr of 72kD and 70kD. Amino acid analysis and N-terminal sequencing of the final product were within 15% of

the expected value.

## EXAMPLE 9

### Antibody Responses of Mice to $NS1_{81}RLf\Delta9$

To evaluate its immunogenicity, purified $NS1_{81}RLf\Delta9$ antigen was inoculated into three strains of mice, C57BL/6 (H-$2^b$); BALB/C (H-$2^d$); and C3H/HEN (H-$2^k$) It has been previously shown that only those mice of the H-$2^b$ haplotype produce antibodies against the repetitive epitope of the P. falciparum circumsporozoite protein. The antigen was injected with Freund's adjuvant and serum samples from immunized animals screened in an enzyme-linked immunoadsorbant assay (ELISA). Control animals were immunized with R32tet32, an antigen containing repeating tetrapeptides of the CS protein. Inoculation of all three mouse strains with $NS1_{81}RLf\Delta9$ resulted in the production of antibody reactive with the repeatless (flanking) region of the circumsporozoite protein. Details and results of the immunogenicity assay follow below.

Each of the three mouse strains was separated into two groups, each group comprising four to five animals. The first group of animals was immunized with $NS1_{81}RLf\Delta9$ and the second group immunized with R32tet32 (J. Young et al., Science, 228:958 (1985)). R32tet32 contains two Xho II fragments, each fragment encoding a peptide having the sequence [$(Asn-Ala-Asn-Pro)_{15}$ (Asn-Val-Asp-Pro)]$_2$. $tet_{32}$ is a 32 amino acid peptide encoded by the tetracycline resistance gene, read out of frame.

$NS1_{81}RLf\Delta9$ antigen, purified according to Example 6, was mixed with complete Freund's adjuvant just prior to administration. Mice (6 to 8 weeks old) were each immunized subcutaneously with 50 $\mu$g. of antigen, administered in a 200 $\mu$l. dose to the right hind quarter. Mice were immunized twice, the first time with a single 200 $\mu$l. dose containing 50 $\mu$g. antigen in complete Freund's adjuvant. Booster injections were given four weeks later according to the same protocol as was used for the first injections except that the antigen was emulsified in incomplete Freund's adjuvant.

Animals were bled at 7 days following the second immunization. Whole blood from all animals within a group was pooled, clotted overnight at 4 °C, and centrifuged to separate the serum which was then stored at -70 °C. An ELISA was used to test the pooled sera for antibody produced against $R32tet_{32}$, $NS1_{81}RLf\Delta9$ or R16CSP. (R16CSP was prepared and purified as described above.)

The "sandwich" ELISA incorporated $R32tet_{32}$, R16CSP and $NS1_{81}RLf\Delta9$ as capture antigens adsorbed to the well walls of a microtitration plate.

The capture antigen was adsorbed to the well walls of the microtitration plate by adding to wells 50 $\mu$l. of a PBS solution containing 0.75 $\mu$g. of capture antigen and 0.2 $\mu$g. of boiled Casein. This solution was prepared by adding 8 $\mu$l. (3.76 $\mu$g.) of capture antigen to 2.5 ml. of a solution consisting of 4 $\mu$l. of a 0.5% boiled Casein solution (described below) to 5 ml. of Dulbecco's phosphate-buffered saline (PBS) (an aqueous solution comprising 0.8% NaCl; 0.217% $Na_2HPO_4$-$7H_2O$; 0.02% $KH_2PO_4$; and 0.02% KCl, having a ph of 7.4).

After overnight incubation at room temperature, the well contents were aspirated and the remaining active binding sites on the plates blocked with a boiled 0.5% Casein solution (5 g/l. Casein (J.T. Baker Chemical Co.); 0.1 g/l. Thimersol (Sigma Chemical Co.); 0.02 g/l. phenol red (Sigma Chemical Co.); 900 ml. PBS, pH 7.4; and 100 ml. 0.1 N NaOH) in admixture (99:1) with 1% Tween 20 (polyoxyethylenesorbitan monolaurate, Sigma Chemical Co.).

Mouse sera samples were diluted 1:100; 1:1000; 1:10,000; 1:100,000 and 1:1,000,000 in a 0.5% boiled Casein solution containing 0.025% Tween 20. The test serum was then added to the well and after 2 hours incubation, the serum removed and the wells washed twice with a PBS solution containing 0.05% Tween 20. An anti-mouse IgG Ab conjugated to peroxidase, diluted 1:2000 with the same diluent used for sera, was then added to the well. After one hour incubation, the well contents were aspirated, washed 3 times with the PBS solution containing 0.05% Tween 20, and a clear peroxidase substrate solution (Kirkegaard & Perry, prepared according to manufacturer's instructions) added. Reaction of the peroxidase with the substrate resulted in the formation of a dark green product, the intensity of the color being proportional to the amount of antibody present in the serum sample. Results were read after 15 minutes at 405 to 414 nanometers using an ELISA plate reader and recorded in Optical Density (0.D.) units.

Results are presented in Figures 1(a) and 1(b) (antibody response in C3H/HEN), Figures 2(a) and 2(b) (antibody response in C57BL/6) and Figures 3(a) and 3(b) (antibody response in BALB/C).

Innoculation of all three mouse strains C3H/HEN (H-$2^k$), BALB/C (H-$2^d$) and C3H/HEN (H-$2^b$) with the $NS1_{81}RLf\Delta9$ antigen resulted in the formation of antibodies which reacted strongly to the repeatless region of the circumsporozoite protein. Reactivity to the $NS1_{81}RLf\Delta9$ capture antigen was only slightly greater than that to R16CSP. (R16CSP capture antigen will detect antibodies only to the repeatless portion of the CS protein whereas the $NS1_{81}RLf\Delta9$ capture antigen permits detection of both anti-$NS1_{81}$ and anti-RLfΔg antibodies). As expected, C3H/HEN mice did not raise an antibody response to $R32tet_{32}$ whereas C57Bl/6 mice did. While significantly weaker (1 log difference) than the antibody response observed in C57BL/6 mice, the antibody response of the BALB/C mice to $R32tet_{32}$ was not expected and is contrary to the negative response reported in the literature for mice of this haplotype to $(NANP)_{40}$(Del Giudice, et al., J. Immunol. 137:2952 (1986) reporting that out of fourteen strains of mice bearing nine different H-2 haplotypes, including BALB/C (H-$2^d$), immunized with $(NANP)_{40}$ without a carrier protein, only H-$2^b$ mice mounted an antibody response against $(NANP)_{40}$. H-$2^d$ mice (BALB/C) did not respond at all. BALB/C mice immunized with $(NANP)_{40}$ coupled to keyhole limpet hemocyanin as a carrier protein did raise anti-$(NANP)_{40}$ antibodies).

## EXAMPLE 9

### Inhibition of Sporozoite Invasion (ISI)

In this study, serum of rabbits immunized with $NS1_{81}RLf\Delta9$ was tested for its ability to inhibit the entry of P. falciparum sporozoites into liver cells, the site of sporozoite development and maturation into the exoerythrocytic stage.

Three mouse strains, BALB/C, C57BL/6 and A/J, immunized with $NS1_{81}RLf\Delta9$, administered in either complete Freund's adjuvant or aluminum hydroxide, developed high antibody titers to the repeatless region of the CS protein. However, sera of these mice was unable to block invasion of sporozoites into cultured human hepatoma cells (HepG2-A16) when assayed according to the Inhibition of Sporozoite Invasion (ISI) assay described in Hollingdale, M.R. et al. J. Immunol, 132(7) 909 (1984).

In contrast, New Zealand white rabbits, immunized with 100 µg. $NSI_{81}RLf\Delta9$, administered in complete Freund's adjuvant at week 0, 3 and 7, elicited elevated antibody titers (albeit lower than that measured in mice) to the repeatless region of the CS protein. However, serum from rabbits immunized with $NS1_{81}RLf\Delta9$ was demonstrated to significantly inhibit (98% in one animal, the average inhibition being about 60%) invasion of hepatoma cells by sporozoites when tested according to the Hollingdale ISI assay.

Sporozoites of a chloroquine resistant strain of P. falciparum (strain 7G8) and a chloroquine sensitive strain of P. falciparum (strain NF54) were each significantly inhibited from entering hepatoma cells by sera from rabbits immunized with $NS1_{81}RLf\Delta9$. Inhibition of hepatoma invasion by sporozoites of P. falciparum strain 7G8 was higher (average 85%) than the ISI determined for strain NF54 (average 60%).

In ISI studies in which normal human hepatocytes were substituted for human hepatoma cells, P. falciparum strain NF54 sporozoites were inhibited (89% inhibition by serum from one rabbit, the average inhibition being about 45%) from entering hepatocytes by sera from rabbits immunized with $NS1_{81}RLf\Delta9$.

These studies suggest the presence of sporozoite neutralizing epitopes on the $NS1_{81}RLf\Delta9$ antigen.

## Claims

1. A polypeptide comprising one or more immunogenic determinants from the first flanking region of a Plasmodium surface protein, one or more immunogenic determinants from the second flanking region and fewer than all or no repeating immunogenic determinants from the repeat domain therebetween.

2. The polypeptide of Claim 1 comprising at least one repeating immunogenic determinant of a Plasmodium surface protein repeat domain between the first and second flanking regions.

3. The polypeptide of Claim 1 comprising substantially all immunogenic determinants of the first flanking region and second flanking region.

4. The polypeptide of Claim 1 wherein the immunogenic determinants of a Plasmodium surface protein are selected from the surface proteins of any of P. falciparum, P. vivax, or P. ovale.

5. The polypeptide of Claim 3 further comprising at least one immunogenic determinant of a

Plasmodium surface protein repeat domain between the first and second flanking regions.

6. The polypeptide of any of Claims 1-5 fused to a carrier protein.

7. The polypeptide of any of Claims 1-6 wherein the surface protein is a circumsporozoite protein.

8. The polypeptide of Claim 6 wherein the carrier protein comprises eighty one N-terminal amino acids of influenza virus non-structural protein 1.

9. The polypeptide of Claim 3 wherein the first flanking region comprises an amino acid sequence corresponding to amino acids 19 (Leu) through 123 (Pro) of P. falciparum CS protein and the second flanking region comprises an amino acid sequence corresponding to amino acids 297 (Gly) through 412 (Asn) of P. falciparum CS protein.

10. The polypeptide of Claim 1 wherein the first flanking region comprises an amino acid sequence corresponding to amino acids 19 (Leu) through 123 (Pro) of P. falciparum CS protein and the second flanking region comprises an amino acid sequence corresponding to amino acids 288 (Asn) through 412 (Asn) of P. falciparum CS protein.

11. The polypeptide of any of Claims 9 and 10 further comprising an immunogenic determinant having the formula $-(Asn-X-Y-Pro)_n-$, wherein X is Ala or Val and Y is Asn or Asp and n is an integer less than 41 when the immunogenic determinant is positioned between the flanking regions and less than 100 when the immunogenic determinant precedes the flanking regions.

12. The polypeptide of Claim 1 having the formula:

$NS1_{1-81}$-Asp-His-Met-Leu-Thr-Asp-Pro-$CS_{19-123}$-$CS_{297-412}$; or

$NS1_{1-81}$-Asp-Hi-Met-Leu-Thr-Asp-Pro-$CS_{19-123}$-Gly-$CS_{288-412}$.

13. The polypeptide of Claim 1 having the formula: $NS1_{1-81}$-Asp-His-Met-Leu-Thr-Asp-Pro-$CS_{19-123}$-$(Asn-X-Y-Pro)_n$-Gly-$CS_{288-412}$, wherein X is Ala or Val and Y is Asn or Asp and n is an integer greater than or equal to one and less than 41.

14. The polypeptide of Claim 1 having the formula:

$NS1_{81}$-Asp-His-Met-Leu-Thr-Asp-Pro-$CS_{19-123}$$(Asn-Ala-Asn-Pro)_n$-Gly-$CS_{288-412}$ wherein n is a an integer greater than or equal to one and less than 41.

15. The polypeptide of Claim 14 wherein n = 2.

16. The polypeptide of Claim 1 having the formula:

$NS1_{81}$-Asp-His-Met-Leu-Thr-Asp-Pro-$CS_{19-123}$-$(Asn-Val-Asp-Pro)_n$Gly-$CS_{288-412}$ wherein n is an integer greater than or equal to one and less than 41.

17. The polypeptide of Claim 1 having the formula:

$NS1_{1-81}$-Asn-X-Y-$Pro)_n$-Asp-His-Met-Leu-Thr-Asp-Pro-$CS_{19-123}$-Gly-$CS_{288-412}$, wherein X is Ala or Val, Y is Asn or Asp and n is an integer greater than or equal to one and less than 100.

· 18. The polypeptide of Claim 1 having the formula:

$NS1_{1-81}$-$(Asn-Ala-Asn-Pro)_n$-Asp-His-Met-Leu-Thr-Asp-Pro-$CS_{19-123}$-Gly-$CS_{288-412}$, wherein n is an integer greater than or equal to one and less than 100.

19. The polypeptide of Claim 18 wherein n = 4.

20. The polypeptide of Claim 1 having the formula:

$NS1_{1-81}$-$(Asn-Val-Asp-Pro)_n$-Asp-His-Met-Leu-Thr-Asp-Pro-$CS_{19-123}$-Gly-$CS_{288-412}$, wherein n is an integer greater than or equal to one and less than 100.

21. The polypeptide of Claim 20 wherein n = 4.

22. An expression vector encoding the polypeptide of Claim 1.

23. The vector of Claim 22 which is an E. coli expression vector;

an E. coli expression vector selected from pMG-1;

$pNS1_{81}RLf\Delta9$; $pNS1_{81}RLfAuth$;

$NS1_{81}RLfAuth + (NANP)_n$ wherein n is an integer greater than or equal to one;

$pNS1_{81}(NANP)_nRLfAuth$, wherein n is an integer greater than or equal to one; and

$pNS1_{81}(NVDP)_n$ RLfAuth, wherein n is an integer greater than or equal to one.

24. A vaccine comprising an immunoprotective amount of a polypeptide according to Claim 1.

25. The use of a polypeptide according to Claim 1 in therapy.

26. The use of a polypeptide according to Claim 1 in the treatment or prophylaxis of infection by Plasmodium sporozoites.

Claims for the following Contracting States: ES and GR

1. A process for preparing a hybrid polypeptide which can stimulate an immune response in an animal to infection by Plasmodium which comprises fusing by synthetic or recombinant DNA techniques one or more immunogenic determinants from the first flanking region of a Plasmodium surface protein to one or

more immunogenic determinants from the second flanking region and fewer than all or no repeating immunogenic determinants from the repeat region therebetween.

2. The process of Claim 1 wherein the hybrid polypeptide which can stimulate an immune response to infection by Plasmodium is prepared by expressing, in a microorganism or cell, a coding sequence comprising a region which codes for an immunogenic determinant from the first flanking region of a Plasmodium surface protein, a region which codes for an immunogenic determinant from the second flanking region, and a region which codes for fewer than all or none of the repeating immunogenic determinants from the repeat domain therebetween.

3. The process of Claim 2 wherein the region which encodes fewer than all or none of the repeating immunogenic determinants from the repeat domain encodes at least one of the repeating immunogenic determinants.

4. The process of Claim 2 wherein the region which encodes the first flanking region and the second flanking region encodes substantially all immunogenic determinants from the first and second flanking regions.

5. The process of Claim 2 wherein the immunogenic determinants are selected from the surface proteins of any of P. falciparum, P. vivax, P. malariae, or P. ovale.

6. The process of Claim 2 wherein the region which encodes fewer than all or none of the repeating immunogenic determinants from the central repeat domain is positioned between the regions encoding the first and second flanking regions.

7. The process of Claim 2 wherein the region encoding the first flanking region encodes an amino acid sequence corresponding to amino acids 19 (Leu) through 123 (Pro) of a P. falciparum circumsporozoite protein and the region encoding the second flanking region encodes an amino acid sequence corresponding to amino acids 297 (Gly) through 412 (Asn) of a P. falciparum circumsporozoite protein.

8. The process of Claim 8 wherein the region encoding the second flanking region encodes an amino acid sequence corresponding to amino acids 288 (Asn) through 412 (Asn) of a P. falciparum oiroumsporozoite protein.

9. The process of Claims 7 and 8 wherein the region encoding fewer than all or none of the repeating immunogenic determinants from the central repeat domain encodes an immunogenic determinant having the formula $(Asn\text{-}X\text{-}Y\text{-}Pro)_n$- wherein X is Ala or Val, Y is Asn or Asp and n is an integer less than 41 when the immunogenic determinant is positioned between the flanking regions and less than 100 when the immunogenic determinant precedes the flanking regions.

Figure 1(a)

ANTIBODY RESPONSE IN C3H/HEN TO NS1R/1

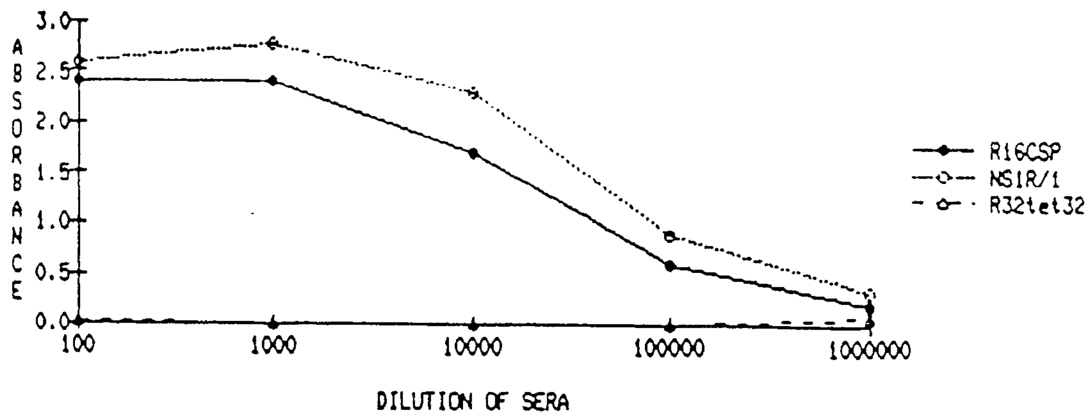


EXP1810G5 04-FEB-88 16:42 Page 1

Figure 1(b)

ANTIBODY RESPONSE IN C3H/HEN TO R32tet32

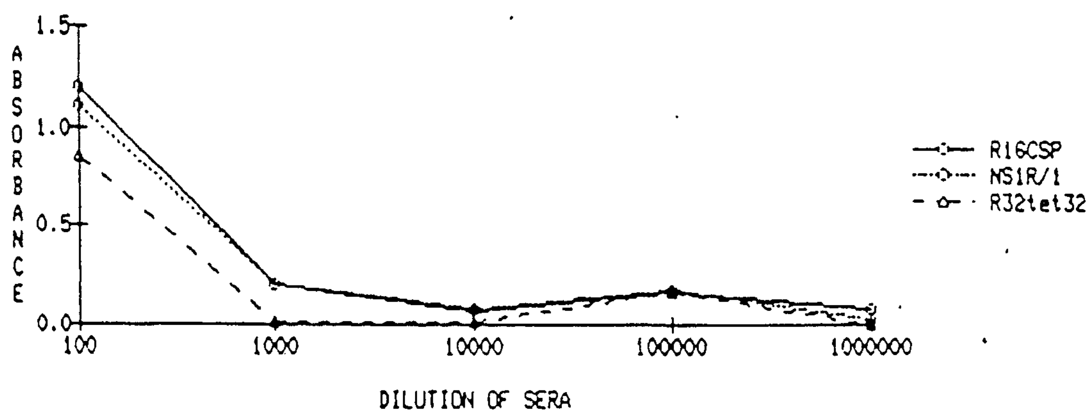

Figure 2(a)

ANTIBODY RESPONSE IN C57BL/6 TO NS1R/1

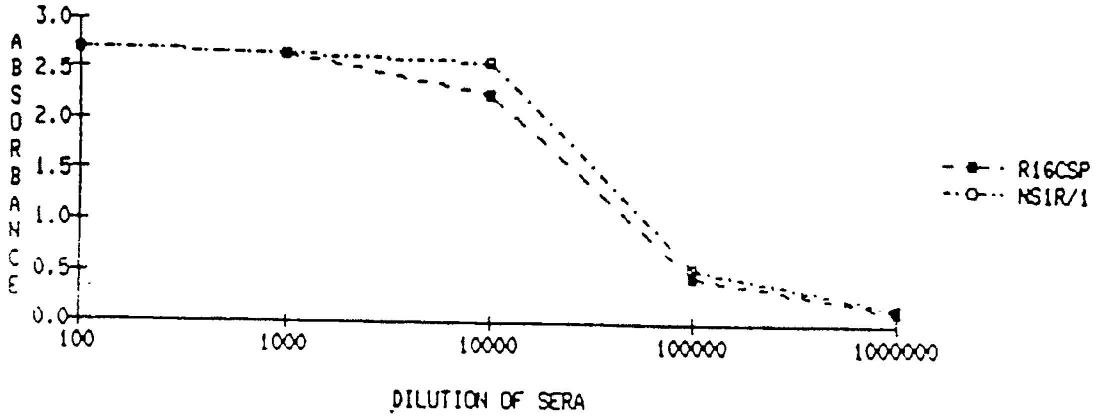


Figure 2(b)

ANTIBODY RESPONSE IN C57BL/6 TO R32tet32

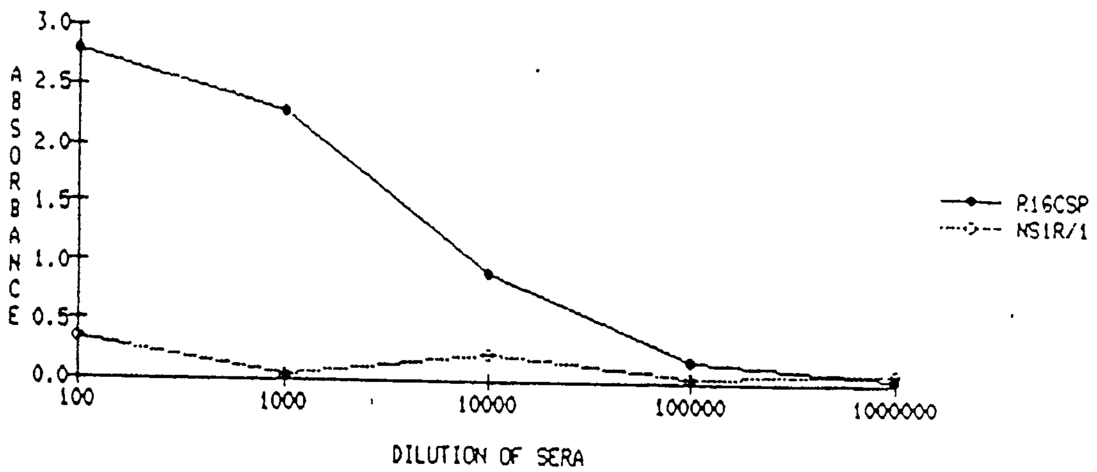

Figure 3(a)

ANTIBODY RESPONSE IN BALB/C TO NS1R/1

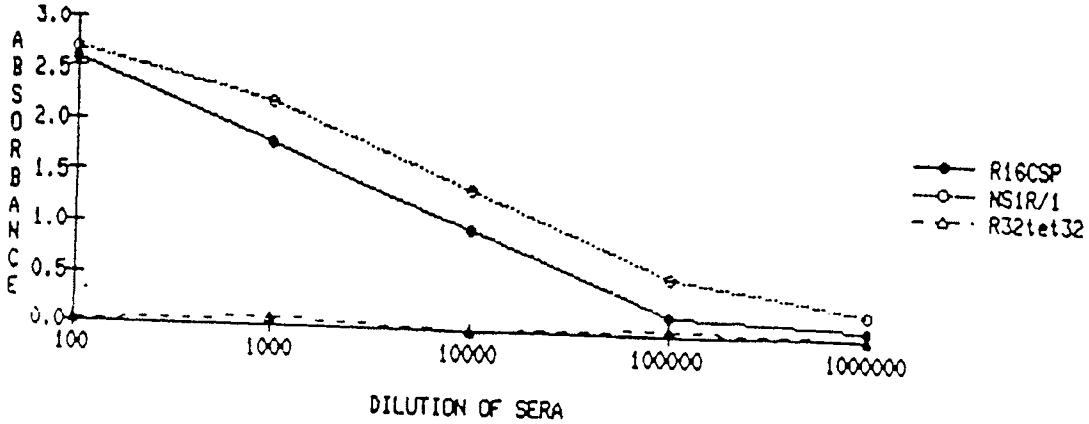


Figure 3(b)

ANTIBODY RESPONSE IN BALB/C TO R32tet32

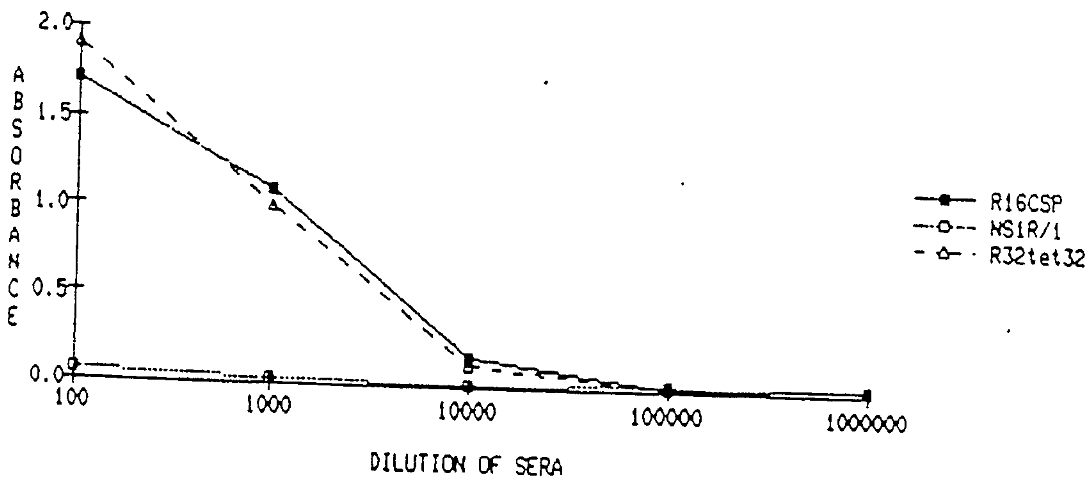

European Patent Office

**PARTIAL EUROPEAN SEARCH REPORT**
which under Rule 45 of the European Patent Convention shall be considered, for the purposes of subsequent proceedings, as the European search report

Application number: EP 90 30 4720

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A- 254 862 (BEHRINGWERKE) | | C 07 K 13/00<br>C 12 N 15/30<br>A 61 K 39/015 |
| | * The whole document * | 1-5,22, 24 | |
| Y | | 6-21, 23 | |
| | -- | | |
| Y | EP-A- 191 748 (SMITH KLINE BECKMAN) | | |
| | * Page 19, line 13 - page 20, line 15 * | 6,8 | |
| | -- | | |
| Y | EP-A- 166 410 (THE UNITED STATES OF AMERICA)<br>* The whole document * | 7,9-21, 23 | |
| | -- | | |
| | ./. | | |

TECHNICAL FIELDS SEARCHED (Int. Cl.4)

C 12 N
A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-24
Claims searched incompletely:
Claims not searched: 25,26
Reason for the limitation of the search:

Method for treatment of the human or animal body by surgery or therapy (see Art. 52(4) of the European Patent Convention).

| Place of search | Date of completion of the search | Examiner |
|---|---|---|
| THE HAGUE | 16-08-1990 | SKELLY |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03.82

European Patent Office

# PARTIAL EUROPEAN SEARCH REPORT

Application number
EP 90 30 4720
-2-

| Category | DOCUMENTS CONSIDERED TO BE RELEVANT<br>Citation of document with indication, where appropriate, of relevant passages | Relevant to claim | CLASSIFICATION OF THE APPLICATION (Int. Cl 4) |
|---|---|---|---|
| A | WO-A-86 01 721 (NEW YORK UNIVERSITY)<br>-- | | |
| A | JOURNAL OF IMMUNOLOGY, vol. 134, no. 5, May 1985, pages 3445-3448, The American Association of Immunologists; U. VERGARA et al.: "Conserved group-specific epitopes of the circumsporozoite proteins revealed by antibodies to synthetic peptides"<br>-- | | |
| | | | TECHNICAL FIELDS SEARCHED (Int Cl 4) |
| A | THE JOURNAL OF BIOLOGICAL CHEMISTRY, vol. 262, no. 14, 15th May 1987, pages 6464-6467, Baltimore, MD, US; V.F. DE LA CRUZ: "Evolution of the immunodominant domain of the circumsporozoite protein gene from plasmodium vivax"<br>---- | | |

EPO Form 1505.3 06.78